# EUROPEAN PATENT SPECIFICATION

(11) **EP 1 795 129 B1**
(45) Date of publication and mention of the grant of the patent: **28.07.2010**
(21) Application number: 06025081.8
(22) Date of filing: 05.12.2006
(51) Int. Cl.: A61B 8/08, G01S 15/89

(54) **Apparatus and method for displaying an ultrasound image**
Vorrichtung und Verfahren zum Anzeigen eines Ultraschallbildes
Appareil et procédé d'affichage d'une image ultrasonore

(30) Priority: 06.12.2005 KR 20050117909
(43) Date of publication of application: 13.06.2007
(73) Proprietor: MEDISON CO., LTD., Kangwon-do 250-870 (KR)
(72) Inventor: Hyun, Dong Gyu, Gangnam-gu, Seoul 135-280 (KR)
(74) Representative: Lorenz, Markus

(56) References cited:
- EP-A- 0 881 506
- US-A- 5 515 856
- DENDY P P ET AL: "Physics for Diagnostic Radiology , Passage" PHYSICS OF DIAGNOSTIC RADIOLOGY, IOP, BRISTOL, US, 15 April 1999 (1999-04-15), pages 344-345, XP002446931

## Description

### BACKGROUND

### 1. Field

The present invention generally relates to ultrasound image processing, and more particularly to an apparatus and method for displaying a 3-dimensional ultrasound image formed based on 2-dimensional ultrasound images.

### 2. Background

An ultrasound diagnostic system has become an important and popular diagnostic tool since it has a wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation techniques such as surgery.

Generally, the ultrasound diagnostic system obtains raw 3D data (e.g., data on a coordinate system (x, y, z)) through a 3D probe regardless of acquisition time by stacking frames over one another at a uniform time interval to form consecutive frames. It then processes the consecutive frames using a 3D rendering technique, thereby producing 3D static images. By using the static 3D images for ultrasound diagnostic purposes, one may easily and accurately observe, diagnose and treat the internal state of a human body without performing any complicated procedures associated with invasive operations. Thus, the static 3D images are widely used. However, the static 3D images are not useful in observing a moving target object in real time, such as a fetus in the uterus.

In order to overcome this shortcoming, a live 3D imaging method and apparatus for providing a live 3D moving image (rather than static 3D images) have been developed. However, a 3-dimensional probe for forming a live 3D ultrasound image is disadvantageous since it is very complex and expensive.

Accordingly, it is highly desirable to form a 3-dimensional ultrasound image without using the 3-dimensional probe.

EP 0 881 506 A2 discloses a three dimensional M-mode imaging system and techniques for acquiring and forming three dimensional ultrasonic M-mode images from ultrasonic data acquired over time from a stationary scanhead. The resultant images exhibit two spatial dimensions and one temporal dimension.

US 5,515,856 discloses a method for generating anatomical M-mode displays for ultrasonic investigation of living biological structures during movement of the structure, for example a heart function, employing an ultrasonic transducer comprises the acquisition of a time series of 2D or 3D ultrasonic images, arranging said time series so as to constitute data sets, providing at least one virtual M-mode line co-registered with said data sets, subjecting said data sets to computer processing on the basis of said at least one virtual M-mode line, whereby interpolation along said at least one virtual M-mode line is effected, and displaying the resulting computed anatomical M-mode display on a display unit

### BRIEF DESCRIPTION OF THE DRAWINGS

Arrangements and embodiments may be described in detail with reference to the following drawings in which like reference numerals refer to like elements and wherein:

FIG. 1 is a schematic block diagram illustrating an ultrasound diagnostic device constructed in accordance with one embodiment of the present invention;

FIG. 2 is a flowchart illustrating a method for displaying an ultrasound image in accordance with one embodiment of the present invention;

FIG. 3 is a schematic diagram illustrating a plurality of 2-dimensional ultrasound images;

FIG. 4 is a schematic diagram illustrating a predetermined number of 2-dimensional ultrasound images to form a 3-dimensional ultrasound image;

FIG. 5 is a schematic diagram illustrating a 3-dimensional ultrasound image formed by superposing the predetermined number of 2-dimensional ultrasound images in accordance with one embodiment of the present invention;

FIG. 6 is a schematic diagram illustrating superposed 2-dimensional ultrasound images;

FIG. 7 is a schematic diagram illustrating an example of setting a flow direction marker and a selection marker on a 3-dimensional ultrasound image in accordance with one embodiment of the present invention;

FIG. 8 is a schematic diagram illustrating an example of displaying a 3-dimensional ultrasound image and a 2-dimensional ultrasound image selected by a selection marker;

FIG. 9 is a schematic diagram illustrating an example of indicating a rotation axis and a time guide line on a 3-dimensional ultrasound image;

FIG. 10 is a schematic diagram illustrating examples of various cutting markers set on a 3-dimensional ultrasound image;

FIG. 11 is a schematic diagram illustrating an example of a cutting marker set on a 3-dimensional ultrasound image; and

FIG. 12 is a schematic diagram illustrating a cross-sectional plane formed along a cutting marker.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

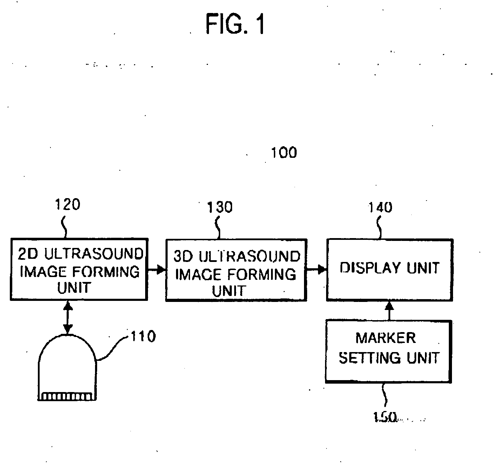

FIG. 1 is a block diagram showing an ultrasound diagnostic device constructed in accordance with the present invention. As shown in FIG. 1, the ultrasound diagnostic device 100 includes a probe 110, a 2-dimensional ultrasound image forming unit 120, a 3-dimensional ultrasound image forming unit 130, a display unit 140 and a marker setting unit 150. The probe 110 transmits ultrasound signals to a target object and receives ultrasound echo signals. The probe 110 may be any probe capable of acquiring a 2-dimensional ultrasound image.

The 2-dimensional ultrasound image forming unit 120 forms a plurality of 2-dimensional ultrasound images having serial numbers based on the ultrasound echo signals. The 2-dimensional ultrasound images may be one of B-mode images, Doppler images and color-mode images. The serial number may be set in an order of acquisition time of the 2-dimensional ultrasound images.

The 3-dimensional ultrasound image forming unit 130 forms a 3-dimensional ultrasound image based on the plurality of 2-dimensional ultrasound images. The 3-dimensional ultrasound image forming unit 130 selects a predetermined number of 2-dimensional ultrasound images and individually renders the selected 2-dimensional ultrasound images. The rendered 2-dimensional ultrasound images are sequentially buffered and then superposed, thereby forming the 3-dimensional ultrasound image.

The 3-dimensional ultrasound image forming unit 130 may provide a transparent 3-dimensional ultrasound image obtained by an appropriate transparency treatment for showing a volume of interest (VOI) in the 3-dimensional ultrasound image. The 3-dimensional ultrasound image or the transparent 3-dimensional ultrasound image formed in the 3-dimensional ultrasound image forming unit 130 is transmitted to the display unit and then displayed on a screen of the display unit 140.

The marker setting unit 150 sets a plurality of markers on the 3-dimensional ultrasound image displayed on the screen of the display unit 140. The markers may represent a rotation axis of the 3-dimensional ultrasound image, a direction along which the 2-dimensional ultrasound images are superposed, and the like. The markers may be set by using control keys provided on a control panel (not shown) of the ultrasound diagnostic device.

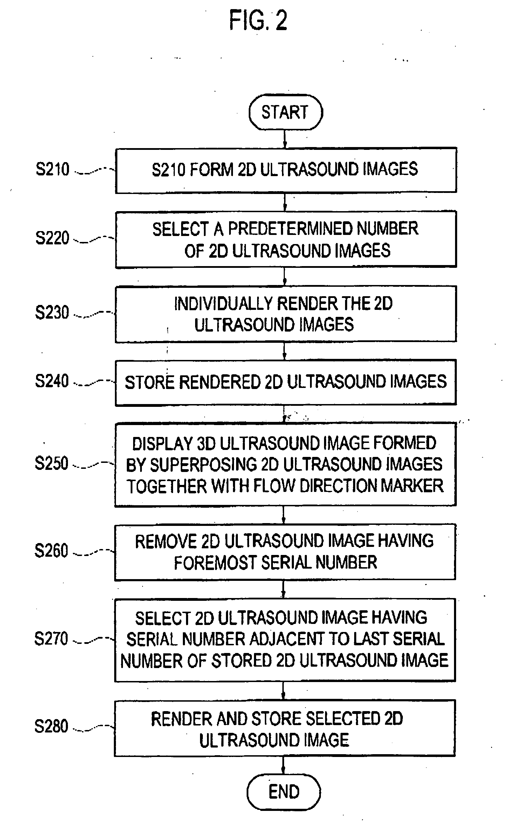

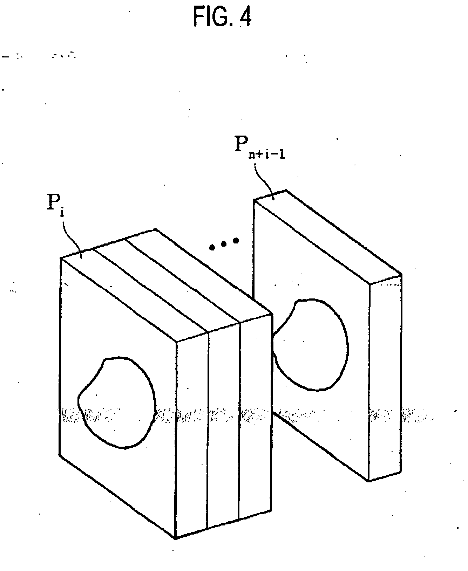

Hereinafter, a method for displaying an ultrasound image will be described in detail with reference to Figs. 2 to 6. Fig. 2 is a flowchart illustrating a method for displaying the ultrasound image in accordance with an embodiment of the present invention.

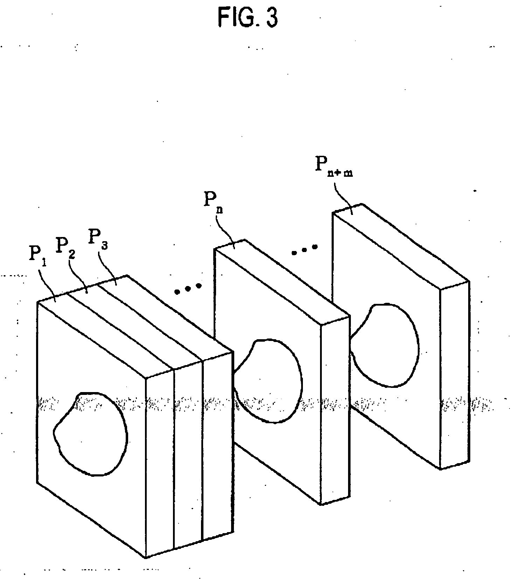

Referring to Fig. 2, the 2-dimensional ultrasound image forming unit 120 forms a plurality of 2-dimensional ultrasound images in real time based on the ultrasound echo signals, which are reflected from a predetermined region of a target object as shown in Fig. 3 at step S210. Serial numbers P1, P2, .... may be assigned to each 2-dimensional ultrasound image. The serial numbers assigned to the 2-dimensional ultrasound images may be set in an order of acquisition time of the 2-dimensional ultrasound images. The 3-dimensional ultrasound image forming unit 130 selects a predetermined number of 2-dimensional ultrasound images having consecutive serial numbers among the plurality of 2-dimensional ultrasound images at step S220.

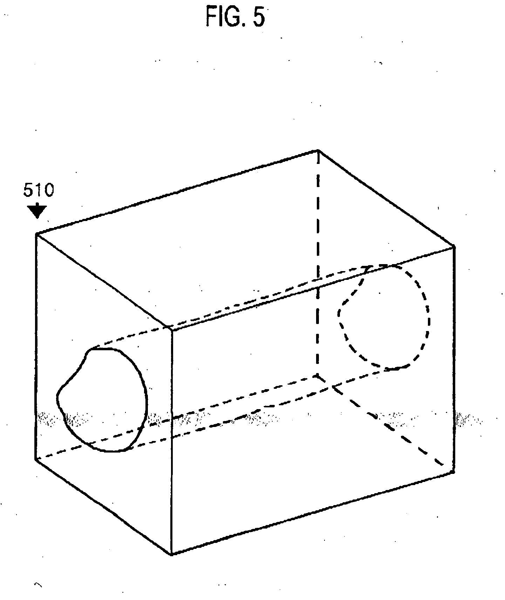

The selected 2-dimensional ultrasound images are individually rendered at step S230. Then, the rendered 2-dimensional ultrasound images are stored at step S240. Thereafter, the stored 2-dimensional ultrasound images are superposed to thereby form and display a 3-dimensional ultrasound image. The 3-dimensional ultrasound image may be displayed together with a flow direction marker indicating a position of a preset 2-dimensional ultrasound image at step S250. Fig. 5 schematically shows a 3-dimensional ultrasound image displayed on a screen. The flow direction marker 510 may be set to indicate a 2-dimensional ultrasound image having a foremost serial number.

Subsequently, a 2-dimensional ultrasound image having a foremost serial number is removed from the stored 2-dimensional ultrasound images at step S260. Then, a 2-dimensional ultrasound image having a serial number adjacent to the last serial number of the stored 2-dimensional ultrasound images is selected at step S270. The 2-dimensional ultrasound image selected at step S270 is rendered and stored at step S280.

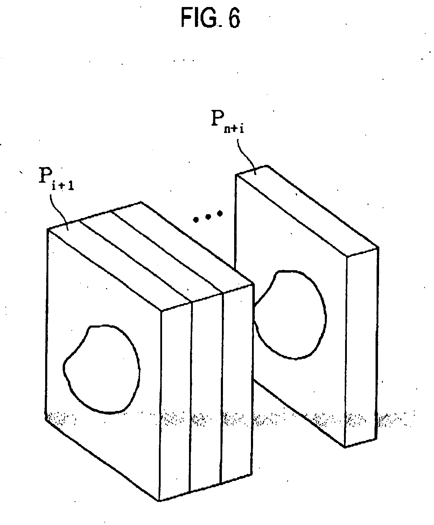

Fig. 6 is a schematic diagram showing the superposed 2-dimensional ultrasound images. The 2-dimensional ultrasound image having the foremost serial number Pi is removed and the 2-dimensional ultrasound image having a serial number Pₙ₊ᵢ adjacent to the last serial number of the stored 2-dimensional ultrasound images is superposed. Steps S240 to S280 are repeatedly performed for the plurality of 2-dimensional ultrasound images formed in the 2-dimensional ultrasound image forming unit, thereby forming and displaying 3-dimensional ultrasound sound images in real time.

Various types of markers may be set together with the flow direction marker on the 3-dimensional ultrasound image displayed on a screen of the display unit 140. The markers may be used to indicate a viewing angle of the 3-dimensional ultrasound image, a position for selecting an arbitrary frame from the frames consisting of the 3-dimensional ultrasound image and a cross-sectional view of the 3-dimensional ultrasound image.

An inverse mode may be applied to the 3-dimensional ultrasound image formed according to the embodiment of the present invention to make the walls of blood vessels or a heart invisible in the 3-dimensional ultrasound image. This is so that a change in the amount of blood in the blood vessel or the heart according to a time change can be easily observed.

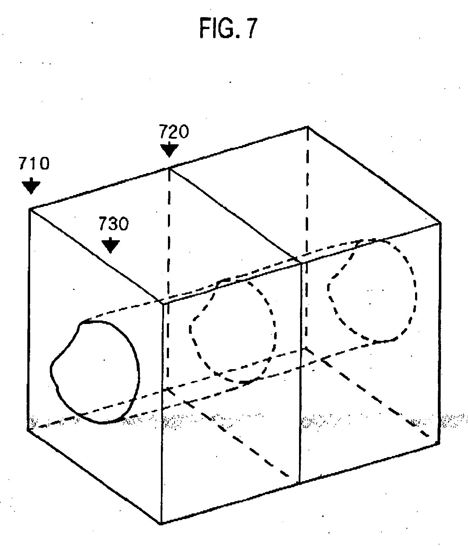

Fig. 7 is a schematic diagram illustrating an example of setting a flow direction marker 710 and a selection marker 720 on the 3-dimensional ultrasound image in accordance with the embodiment of the present invention. As shown in Fig. 7, the flow direction marker 710 indicating a 2-dimensional ultrasound image having a foremost serial number and the selection marker 720 indicating an arbitrary 2-dimensional ultrasound image are set on the 3-dimensional ultrasound image. A direction, along which the 2-dimensional ultrasound images are superposed, may be identified through the flow direction marker 710. If the 2-dimensional ultrasound image having the foremost serial number is removed from the 3-dimensional ultrasound image, then the flow direction marker 710 automatically indicates a 2-dimensional ultrasound image having a next serial number.

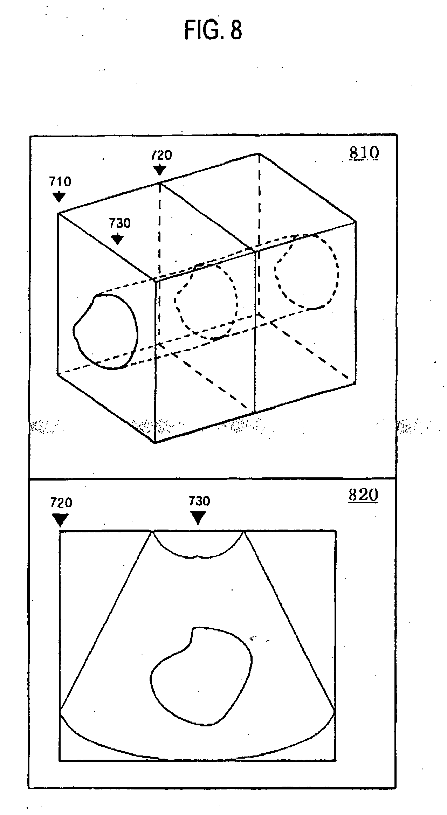

The selection marker 720 may be replaced with the flow direction marker 710. For example, if a user inputs a setup instruction of the selection marker, then the marker setting unit 150 sets the flow direction marker 710 displayed on the 3-dimensional ultrasound image as a selection marker 720. If the user inputs a moving instruction of the selection marker 720, then the marker setting unit 150 moves the position of the selection marker 720 on the 3-dimensional ultrasound image in response to the moving instruction. Further, the screen of the display unit 140 is divided into a first region 810 and a second region 820, as shown in Fig. 8. The 3-dimensional ultrasound image is displayed on the first region 810 and a 2-dimensional ultrasound image position at the moved selection marker 720 is displayed on the second region 820. Further, an image direction marker 730 indicating a direction of a target object in the 3-dimensional ultrasound image may be displayed.

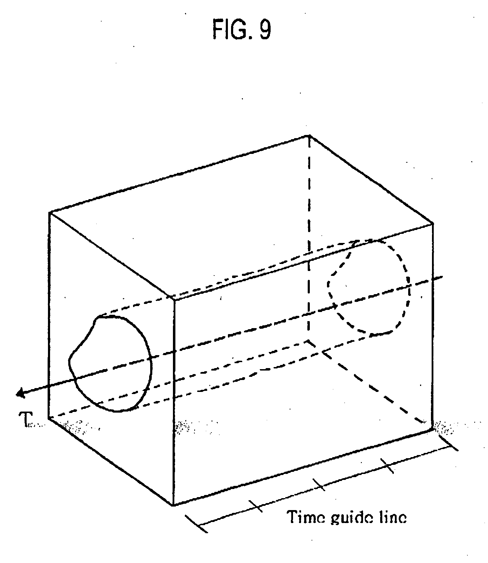

Fig. 9 is a schematic diagram illustrating an example of setting a rotation marker on the 3-dimensional ultrasound image in accordance with the embodiment of the present invention. A time axis may be used as a rotation marker T. The rotation axis T is used to rotate the 3-dimensional ultrasound image, thereby displaying the 3-dimensional ultrasound image in various directions. Also, if the rotation axis corresponds to the time axis, then a time guide line indication for a moving period of the target object such as a heart may be provided on the screen. It is preferable that the time guide line is displayed in parallel with an array direction of the 2-dimensional ultrasound images.

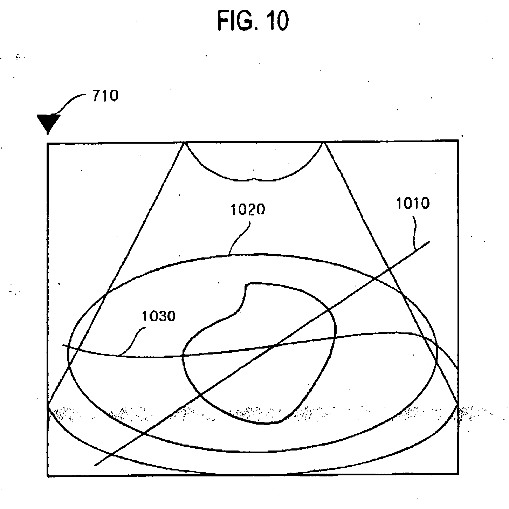

Fig. 10 is a schematic diagram showing an example of setting various cutting markers for showing cross-sectional views of the 3-dimensional ultrasound image. If the user inputs a cutting marker setting instruction, the marker setting unit 160 sets the cutting marker on the 2-dimensional ultrasound image displayed on the second region 820 so that the 2-dimensional ultrasound image is divided into at least two regions. Thereafter, if the user selects one of the regions by using an indicator (not shown), a cross-sectional plane image corresponding to the selected region is displayed on the screen. As shown in Fig. 10, the cutting marker may include a line cutting marker 1010, a cure cutting marker 1020, a free line cutting marker 1030 and the like.

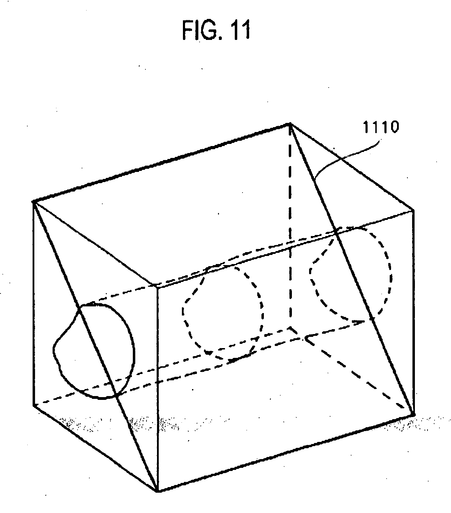

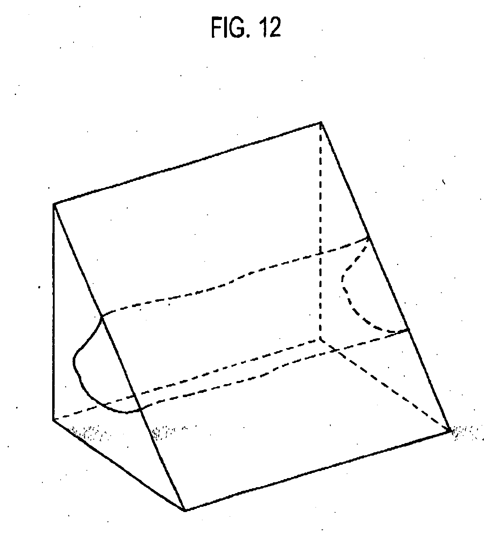

Fig. 11 shows an example of indicating a line cutting marker 1110 on the 3-dimensional ultrasound image. Fig. 12 shows a cross-sectional plane obtained by cutting the 3-dimensional ultrasound image along the line cutting marker 1110.

As mentioned above, the 3-dimensional ultrasound image is formed by superposing the 2-dimensional ultrasound images in accordance with one embodiment of the present invention. This is so that the 3-dimensional ultrasound image can be provided without using an expensive 3-dimensional probe. Also, as the 3-dimensional ultrasound image is formed by individually rendering the 2-dimensional ultrasound images, a time for forming the 3-dimensional ultrasound image can be reduced, thereby providing the 3-dimensional ultrasound image in real time.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A method for displaying an ultrasound image, comprising:
a) forming and storing a plurality of sequential 2-dimensional ultrasound images based on ultrasound echo signals reflected from a target object, each of said sequential 2-dimensional ultrasound images being assigned a serial number;
b) selecting N numbers of 2-dimensional ultrasound images having consecutive serial numbers;
c) individually rendering the selected 2-dimensional ultrasound images and superposing the N number of rendered 2-dimensional ultrasound images to form a 3-dimensional ultrasound image;
d) forming a flow direction marker indicating a first 2-dimensional ultrasound image from the N numbers of 2-dimensional ultrasound images on the 3-dimensional ultrasound image;
e) displaying the 3-dimensional ultrasound image together with the flow direction marker on a screen;
f) removing the first 2-dimensional ultrasound image from the N numbers of 2-dimensional ultrasound images;
g) selecting a (N+1)^{th} 2-dimensional ultrasound image, rendering the selected (N+1)^{th} 2-dimensional ultrasound image and superposing the selected 2-dimensional ultrasound image to the superposed 2-dimensional ultrasound images; and
h) repeating the steps c) to g) as many as a predetermined number.

2. The method of Claim 1, after forming the flow direction marker at step d), further comprising.
d11) receiving a moving instruction from a user;
d12) moving the flow direction marker in response to the moving instruction; and
d13) displaying the 3-dimensional ultrasound image on a first region of the screen and a 2-dimensional ultrasound image positioned at the moved flow direction marker on a second region of the screen.

3. The method of Claim 1, wherein the serial numbers are determined in an order of acquisition time of the 2-dimensional ultrasound images.

4. The method of Claim 3, wherein the flow direction marker is set to indicate a first 2-dimensional ultrasound image among the superposed 2-dimensional ultrasound images.

5. The method of Claim 4, wherein a time guide line parallel to a superposing direction of the 2-dimensional ultrasound images is displayed together with the 3-dimensional ultrasound image.

6. The method of Claim 1, wherein the 3-dimensional ultrasound image is inversed and the inversed 3-dimensional ultrasound image is displayed.

7. The method of Claim 1, after forming the 3-dimensional ultrasound image at step d), further comprising:
d21) receiving a cutting marker setting instruction from a user;
d22) setting at least one cutting marker on the 3-dimensional ultrasound image, thereby dividing the 3-dimensional ultrasound image into a plurality of regions:
d23) selecting one of regions; and
d24) displaying the selected region.

8. An apparatus (100) of displaying an ultrasound image, comprising: a probe (110) for transmitting ultrasound signals into a target object and receiving ultrasound echo signals; and a 2-dimensional ultrasound image forming unit (120) for forming a plurality of 2-dimensional ultrasound image based on the ultrasound image signals, each of the plurality of 2-dimensional ultrasound images being assigned a serial number,
**characterized by** further comprising:
a 3-dimensional ultrasound image forming unit (130) for selecting consecutive N numbers of 2-dimensional ultrasound images, individually rendering the selected 2-dimensional ultrasound images and superposing the N numbers of 2-dimensional ultrasound images, thereby forming a 3-dimensional ultrasound image, wherein the 3-dimensional ultrasound image forming unit (130) removes a first 2-dimensional ultrasound image from the N numbers of 2-dimensional ultrasound images, selects a (N+1)^{th} 2-dimensional ultrasound image, and forms the 3-dimensional ultrasound image by superposing the selected 2-dimensional ultrasound images to the superposed 2-dimensional ultrasound images;
a marker setting unit (150) for setting at least one marker on the 3-dimensional ultrasound image; and
a displaying unit (140) for displaying the 3-dimensional ultrasound image and the marker.

9. The apparatus of Claim 8, wherein the serial numbers are determined in an order of acquisition time of the 2-dimensional ultrasound images.

10. The apparatus of Claim 9, wherein the marker includes a flow direction marker indicating a first 2-dimensional ultrasound image among the superposed 2-dimensional ultrasound images and a cutting marker indicating a cutting position on the 3-dimensional ultrasound image.

## Patentansprüche

1. Verfahren zum Anzeigen eines Ultraschallbildes, welches Folgendes aufweist:
a) Erstellen und Speichern einer Vielzahl von sequenziellen 2-dimensionalen Ultraschallbildern basierend auf von einem Zielobjekt reflektierten E-chosignalen, wobei jedem der sequenziellen 2-dimensionalen Ultraschallbilder eine Seriennummer zugeteilt wird;
b) Auswählen einer Anzahl N von 2-dimensionalen Ultraschallbildern, welche fortlaufende Seriennummern haben;
c) Individuell Rendern der ausgewählten 2-dimensionalen Ultraschallbilder und Überlagern der N Anzahl von gerenderten 2-dimensionalen Ultraschallbildern, um ein 3-dimensionales Ultraschallbild zu erstellen;
d) Erstellen einer Strömungsrichtungsmarkierung, welche ein erstes 2-dimensionales Ultraschallbild von der Anzahl N von 2-dimensionalen Ultraschallbildern auf dem 3-dimensionalen Ultraschallbild anzeigt;
e) Anzeigen des 3-dimensionalen Ultraschallbildes zusammen mit der Strömungsrichtungsmarkierung auf einem Bildschirm;
f) Entfernen des ersten 2-dimensionalen Ultraschallbildes von der Anzahl N von 2-dimensionalen Ultraschallbildern;
g) Auswählen eines (N+1)-ten 2-dimensionalen Ultraschallbildes, Rendern des ausgewählten (N+1)-ten 2-dimensionalen Ultraschallbildes und Überlagern des ausgewählten 2-dimensionalen Ultraschallbildes mit den überlagerten 2-dimensionalen Ultraschallbildern; und
h) Wiederholen der Schritte c) bis g) bis zu einer vorbestimmten Anzahl.

2. Verfahren nach Anspruch 1, welches nach dem Erstellen der Strömungsrichtungsmarkierung an dem Schritt d) Folgendes aufweist:
d11) Empfangen einer Bewegungsanweisung von einem Benutzer;
d12) Bewegen der Strömungsrichtungsmarkierung als Reaktion auf die Bewegungsanweisung; und
d13) Anzeigen des 3-dimensionalen Ultraschallbilds auf einem ersten Bereich des Bildschirms und eines 2-dimensionalen Ultraschallbilds, welches an der bewegten Strömungsrichtungsmarkierung positioniert ist, auf einem zweiten Bereich des Bildschirms.

3. Verfahren nach Anspruch 1, wobei die Seriennummern in einer Reihenfolge der Erfassungszeit der 2-dimensionalen Ultraschallbilder festgelegt werden.

4. Verfahren nach Anspruch 3, wobei die Strömungsrichtungsmarkierung so eingestellt ist, dass sie ein erstes 2-dimensionales Ultraschallbild unter den überlagerten 2-dimensionalen Ultraschallbildern anzeigt.

5. Verfahren nach Anspruch 4, wobei eine Zeitrichtlinie parallel zu einer überlagerten Richtung der 2-dimensionalen Ultraschallbilder zusammen mit dem 3-dimensionalen Ultraschallbild angezeigt wird.

6. Verfahren nach Anspruch 1, wobei das 3-dimensionale Ultraschallbild invertiert und das invertierte 3-dimensionale Ultraschallbild angezeigt wird.

7. Verfahren nach Anspruch 1, welches nach dem Bilden des 3-dimensionalen Ultraschallbilds an dem Schritt d) des weiteren Folgendes aufweist:
d21) Empfangen einer Anweisung zum Setzen einer Schneidmarkierung von einem Benutzer;
d22) Setzen wenigstens einer Schneidmarkierung auf dem 3-dimensionalen Ultraschallbild, wodurch das 3-dimensionale Ultraschallbild in eine Vielzahl von Bereichen unterteilt wird;
d23) Auswählen von einem der Bereiche; und
d24) Anzeigen des ausgewählten Bereichs.

8. Vorrichtung (100) zum Anzeigen eines Ultraschallbilds, welche Folgendes aufweist:
einen Messkopf (110) zum Übertragen von Ultraschallsignalen in ein Zielobjekt und Empfangen von Ultraschallechosignalen; und eine 2-dimensionales Ultraschallbild-Erstelleinheit (120) zum Erstellen einer Vielzahl von 2-dimensionalen Ultraschallbildern basierend auf den Ultraschallbildsignalen,
wobei jedem der Vielzahl von 2-dimensionalen Ultraschallbildern eine Seriennummer zugeteilt ist,
**dadurch gekennzeichnet, dass** sie des weiteren Folgendes aufweist:
eine 3-dimensionale Ultraschallbild-Erstelleinheit (130) zum Auswählen einer Anzahl N von aufeinanderfolgenden 2-dimensionalen Ultraschallbildern, individuellen Rendern der ausgewählten 2-dimensionalen Ultraschallbilder und Überlagern der Anzahl N von 2-dimensionalen Ultraschallbildern und dadurch Erstellen eines 3-dimensionalen Ultraschallbilds, wobei die 3-dimensionale Ultraschallbild-Erstelleinheit (130) ein erstes 2-dimensionales Ultraschallbild von der Anzahl N von 2-dimensionalen Ultraschallbildern entfernt, ein (N+1)-tes 2-dimensionales Ultraschallbild auswählt und das 3-dimensionale Ultraschallbild durch Überlagern der ausgewählten 2-dimensionalen Ultraschallbilder auf die überlagerten 2-dimensionalen Ultraschallbilder erstellt;
eine Markiersetzeinheit (150) zum Setzen von wenigstens einer Markierung auf dem 3-dimensionalen Ultraschallbild; und
eine Anzeigeeinheit (140) zum Anzeigen des 3-dimensionalen Ultraschallbilds und der Markierung.

9. Vorrichtung nach Anspruch 8, wobei die Seriennummern in einer Reihenfolge der Erfassungszeit der 2-dimensionalen Ultraschallbilder festgelegt werden.

10. Vorrichtung nach Anspruch 9, wobei die Markierung eine Strömungsrichtungsmarkierung, welche ein erstes 2-dimensionales Ultraschallbild zwischen den überlagerten 2-dimensionalen Ultraschallbildern und eine Schneidmarkierung beinhaltet, welche eine Schneidposition auf dem 3-dimensionalen Ultraschallbild anzeigt.

## Revendications

1. Procédé pour afficher une image ultrasonore, comprenant :
a) la formation et le stockage d'une pluralité d'images ultrasonores bidimensionnelles successives basées sur des signaux échos d'ultrasons réfléchis par un objet cible, chacune desdites images bi-dimensionnelles successives recevant un numéro de série ;
b) la sélection de N numéros d'images ultrasonores bidimensionnelles ayant des numéros de série consécutifs ;
c) le rendu individuellement des images ultrasonores bidimensionnelles sélectionnées et la superposition du nombre N d'images ultrasonores bidimensionnelles rendues pour former une image ultrasonore tridimensionnelle;
d) la formation d'un marqueur de sens de passage indiquant une première image ultrasonore bidimensionnelle parmi les N numéros d'images ultrasonores bidimensionnelles sur l'image ultrasonore tridimensionnelle ;
e) l'affichage de l'image ultrasonore tridimensionnelle avec le marqueur de sens de passage sur un écran ;
f) le retrait de la première image ultrasonore bidimensionnelle des N nombres d'images ultrasonores bidimensionnelles :
g) la sélection d'une (N+1)^{ème} image ultrasonore bidimensionnelle, le rendu de la (N+1)^{ème} image ultrasonore bidimensionnelle et la superposition de l'image ultrasonore bidimensionnelle sélectionnée aux images ultrasonores bidimensionnelles superposées ; et
h) la répétition des étapes c) à g) un nombre de fois prédéterminé.

2. Procédé selon la revendication 1, comprenant en outre après la formation du marqueur de sens de passage à l'étape d):
d11) la réception d'une instruction de déplacement à partir d'un utilisateur;
d12) le déplacement du marqueur de sens de passage en réponse à l'instruction de déplacement ; et
d13) l'affichage de l'image ultrasonore tridimensionnelle sur une première région de l'écran et d'une image ultrasonore bidimensionnelle positionnée à l'endroit du marqueur de sens de passage sur une deuxième région de l'écran.

3. Procédé selon la revendication 1, dans lequel les numéros de série sont déterminés dans un ordre de temps d'acquisition des images ultrasonores bidimensionnelles.

4. Procédé selon la revendication 3, dans lequel le marqueur de sens de passage est réglé pour indiquer une première image ultrasonore bidimensionnelle parmi les images ultrasonores bidimensionnelles superposées.

5. Procédé selon la revendication 4, dans lequel une ligne de guidage de temps parallèle à une direction de superposition des images ultrasonores bidimensionnelles est affichée avec l'image ultrasonore tridimensionnelle.

6. Procédé selon la revendication 1, dans lequel l'image ultrasonore tridimensionnelle est inversée et l'image ultrasonore tridimensionnelle inversée est affichée.

7. Procédé selon la revendication 1, comprenant en outre, après la formation de l'image ultrasonore tridimensionnelle à l'étape d), :
d21) la réception d'un utilisateur d'une instruction de réglage de marqueur de coupe ;
d22) le réglage d'au moins un marqueur de coupe sur l'image ultrasonore tridimensionnelle, en divisant par ce moyen l'image ultrasonore tridimensionnelle en une pluralité de régions ;
d23) la sélection d'une des régions ; et
d24) l'affichage de l'image sélectionnée.

8. Dispositif (100) d'affichage d'une image ultrasonore, comprenant une sonde (110) destinée à transmettre des signaux ultrasonores dans un objet cible et recevoir des signaux échos ultrasonores, et une unité de formation d'images ultrasonores bidimensionnelles (120) pour former une pluralité d'images ultrasonores bidimensionnelles basées sur les signaux d'images ultrasonores, chacune des images ultrasonores bidimensionnelles recevant un numéro de série,
**caractérisé en ce qu'**il comprend en outre :
une unité de formation d'images ultrasonores tridimensionnelles (130) destinée à sélectionner N numéros successifs consécutifs d'images ultrasonores bidimensionnelles, à rendre individuellement les images ultrasonores bidimensionnelles sélectionnées, et à superposer les N numéros d'images ultrasonores bidimensionnelles, en formant par ce moyen une image ultrasonore tridimensionnelle, dans lequel l'unité de formation d'image ultrasonore tridimensionnelle (130) enlève une première image ultrasonore bidimensionnelle des N numéros d'images ultrasonores bidimensionnelles, sélectionne une (N+1)^{ème} image ultrasonore bidimensionnelle et forme une image ultrasonore tridimensionnelle en superposant les images ultrasonores bidimensionnelles sélectionnées aux images ultrasonores bidimensionnelles superposées ;
une unité de réglage de marqueur (150) destinée à régler au moins un marqueur sur l'image ultrasonore tridimensionnelle ; et
une unité d'affichage (140) destinée à afficher l'image ultrasonore tridimensionnelle et le marqueur.

9. Dispositif selon la revendication 8, dans lequel les numéros de série sont déterminés dans un ordre de temps d'acquisition des images ultrasonores bidimensionnelles.

10. Dispositif selon la revendication 9, dans lequel le marqueur comprend un marqueur de sens de passage qui indique une première image ultrasonore bidimensionnelle parmi les images ultrasonores bidimensionnelles superposés et un marqueur de coupe qui indique une position de coupe sur l'image ultrasonore tridimensionnelle.
